(19) Europäisches Patentamt
European Patent Office
Office européen des brevets

(11) Publication number: 0 268 245
A2

(12) EUROPEAN PATENT APPLICATION

(21) Application number: 87116885.2

(51) Int. Cl.[4]: C07D 461/00

(22) Date of filing: 16.11.87

(30) Priority: 17.11.86 JP 271937/86

(43) Date of publication of application:
25.05.88 Bulletin 88/21

(84) Designated Contracting States:
CH DE FR GB IT LI

(71) Applicant: SANWA KAGAKU KENKYUSHO CO., LTD.
No. 35, Higashi-sotobori-cho
Higashi-ku Nagoya-shi Aichi-ken(JP)

(72) Inventor: Kurono, Masayasu
3-6-7, Sasao-nishi Toin-cho
Inabe-gun Mie-ken(JP)
Inventor: Usui, Toshinao
5-45, Minami-uzura
Gifu-shi Gifu-ken(JP)

(74) Representative: Wächtershäuser, Günter, Dr.
Tal 29
D-8000 München 2(DE)

# (54) Process for the preparation of eburnamenine oxime derivatives.

(57) A process for the preparation of an eburnamenine oxime derivative of the formula

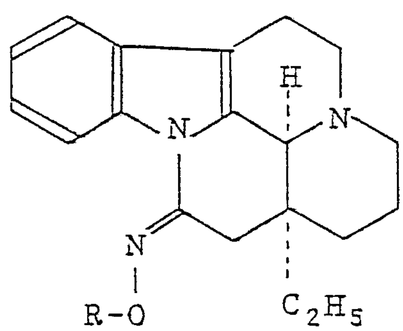

(I)

wherein R is an alkyl group, alkoxyalkyl group, oxylanylalkyl group, aralkyl group, aryl group, heteroaryl group,

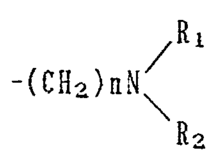 group or 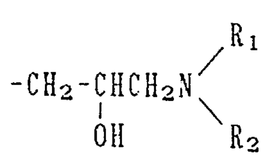 group,

each of $R_1$ and $R_2$ is same or different and represents hydrogen atom, an alkyl group having 1 to 4 carbon atoms, aryl group or aralkyl group, or $R_1$ and $R_2$ may form a substituted or unsubstituted heterocyclic radical together with the neighboring nitrogen atom, and n is an integer of 2 or 3,
which comprises a step of reacting aburnamenine thione of the formula

EP 0 268 245 A2

Xerox Copy Centre

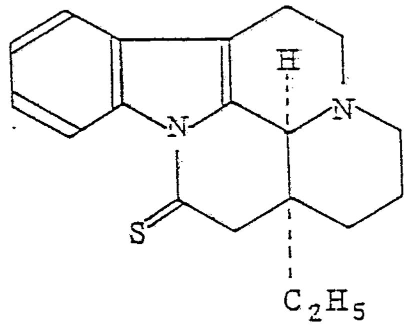

(II)

with a compound of the formula
$R\text{-}ONH_2$
wherein R represents the meaning as referred to.

## PROCESS FOR THE PREPARATION OF EBURNAMENINE OXIME DERIVATIVES

The present invention relates to a process for the preparation of eburunamenine oxime derivatives usable as an effective ingredient for medicines.

It has been known that various derivatives of eburnamenine oxime have cerebral vasodilating and metabolic activating actions and thus those are useful for curing cerebral apoplexy, cerebral arteriosclerosis, hypertensive cerebral circulatory insuffucuency, retension defect, cephalagia and other diseases due to cerebral circulatory disturbances and/or reduction of oxygen utilizing function.

Hitherto, the eburunamenine oxime derivatives have been prepared through steps of converting eburunemenine thione, as the starting material, into eburunemenine oxime, alkylating the oxime and then further modifying the resulting compound to look out a substance having more excellent pharmacological action [see, for instance, Jap. Unexamined Pat. Appln. Gazette Nos. 12687/1986 and 200987/1986 (Jap. Pat. Appln. Nos. 132926/1984 and 41235/1985), corresponding to U.S. Pat. Appln. Ser. No. 740775 and European Pat. Appln. No 85304529.2].

Each of the processes as disclosed in said Japanese publications has the advantage in yield but steps for synthesizing a desired compound becomes longer, so that it had the disadvantage of rising up of its manufacturing cost.

An object of the present invention is, therefore, to provide a process for the preparation of eburunamenine oxime derivatives, which is suitable for a large scale production thereof with a reasonable cost.

A specific object of the invention is to provide the process starting from eburunamenine thione and converting same into a desired specific compound by one step only.

According to the invention, the above objects and other objects to be appreciated by fully understanding the invention can be attained by a process for the preparation of an eburnamenine oxime derivative of the formula

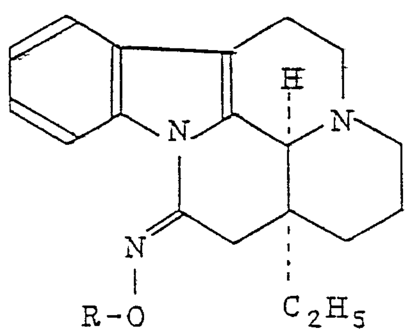

(I)

wherein R is an alkyl group, alkoxyalkyl group, oxylanylalkyl group, aralkyl group, aryl group, heteroaryl group,

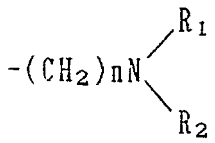 group or 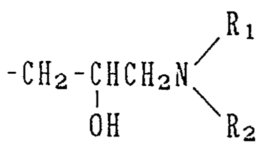 group,

each of $R_1$ and $R_2$ is same or different and represents hydrogen atom, an alkyl group having 1 to 4 carbon atoms, aryl group or aralkyl group, or $R_1$ and $R_2$ may form a substituted or unsubstituted heterocyclic radical together with the neighboring nitrogen atom, and n is an integer of 2 or 3,

which comprises a step of reacting eburnamenine thione of the formula

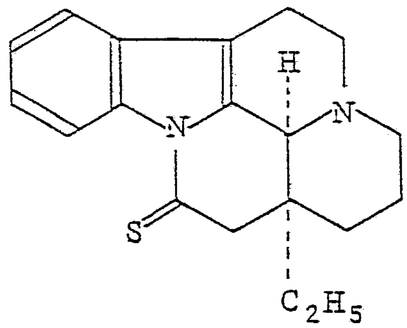

(II)

with a compound of the formula

R-$ONH_2$ (III)

wherein R represents the meaning as referred to.

In the process for the invention, the reaction between the compounds II and III may be carried out in the absence of any solvent, but it is preferable to use a solvent. As the solvent, for instance methanol, ethanol, isopropanol, tetrahydrofuran, dioxane, acetonitrile, dimethylsulfoxide, carbon tetrachloride, chloroform, methylene chloride, triethylamine, pyridine, benzene, toluene or any mixture thereof may be listed. It is preferable to use the compound III in molar amount of 1.0 to 5.0 time to the eburunamenine thione (compound II) and to react the compounds for 0.5 to 6.5 hours at a temperature between 20 to 100°C. Further, mercuric chloride, cupric chloride, cupric bromide, manganese chloride, stannous chloride, mercuric acetate, cupric acetate or the like organis or inorganic salt of heavy metals, or a complex thereof may be added as a condensation agent. Moreover, the reaction may be carried out in the presence of an organic or inorganic salt or an alkali metal, for instance sodium hydrogen carbonate, sodium carbonate, potassium hydrogen carbonate, potassium carbonate, sodium acetate, potassium acetate, sodium hydrogen phosphate, potassium hydrogen phosphate, potassium phosphate or the like, or an organic or inorganic salt of an alkaline earth metal, for instance calcium carbonate, strontium carbonate, calcium acetate, strontium acetate, calcium phosphate, strontium phosphate or the like.

It was found out through various experiments that the optimum synthesizing condition lies in using the compound III in molar amount of 1.5 times to the eburunamenine thione (compound II) and heating the compounds in the presence of mercuric chloride and potassium carbonate, each of which is added in equimolar amount with the compound II and in the mixed solvent of chloroform and acetonitrile, which solvent mixture is in refluxed state.

The invention will now be further explained with reference to Examples on the preparation of eburunamenine oxime derivative.

Example 1

(3α, 16α)-Eburunamenine-14(15H)-one 0-[2-N,N-diethylamino)-ethyl]oxime

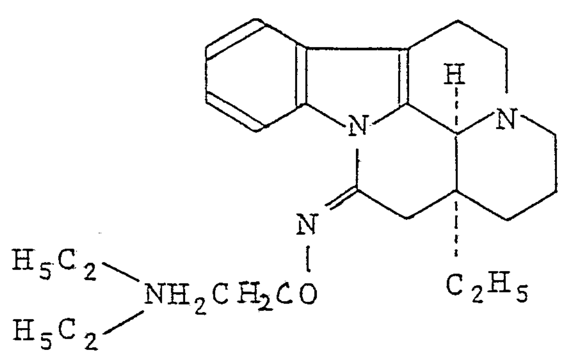

A mixture of (3α, 16α)-Eburunamenine-14(15H)-thione (80.3mg, 0.259 mmol), 2-(N,N-diethylamino)-ethoxyamine (51.3mg, 0.389mmol), mercuric chloride (70.4mg, 0.259mmol), potassium carbonate (35.8mg, 0.259mmol), acetonitrile (2.00ml, 38.2mmol) and chloroform (2.00ml, 24.8mmol) was refluxed for 2 hours under vigorous stirring. After cooling the reaction mixture, the undissolved substance was filtered off and the

filtrate was evaporated in vacuo. The residue was purified by chromatography on silica gel column and with chloroform/methanol (9/1) as eluent to give 103mg (97.3%) of the desired compound as pale yellow oil.
Mass spectrum (m/z) : 408
NMR spectrum ($CDCl_3$) δppm :
0.92 (3H, broad t, J = 7.0Hz, $-CH_2\underline{CH_3}$)
0.95 (6H, t, J = 7.0Hz, $N-CH_2-\underline{CH_3}$ x 2)
1.0 - 3.6 (14H, m, $CH_2$ x 7)
2.62 (4H, q, J = 7.0Hz, $N\underline{CH_2}CH_3$ x 2)
2.84 (2H, t, J = 6.0Hz, $-CH_2N-$)
3.83 (1H, broad s, $C_{3\alpha}$-H)
4.22 (2H, t, J = 6.0Hz, $-OCH_2-$)
7.0 - 7.6 (3H, m, Ar-H)
8.2 - 8.5 (1H, m, Ar-H)
IR spectrum ($\nu^{neat}_{max}$) $cm^{-1}$ :
3100 - 2800 (C-H), 1640, 1615 (C=C, C=N), 1460, 1405, 1050, 745

Examples 2 to 9

The reaction of (3α, 16α)-eburunamenine-14(15H)-thione (compound A) with 2-(N,N-diethylamino)-ethoxyamine (compound B) was carried out by the similar procedure in Example 1 but by changing the reaction condition in various manner to obtain (3α, 16α)-eburunamenine-14(15H)-one 0-[2-(N,N-diethylamino)ethyl]oxime (compound C).

The selected reaction condition for and result in each case were shown in the following Table.

TABLE

| Example | Compound A (mol) | Compound B (mol) | Heavy metal salt (mol) | $K_2CO_3$ (mol) | $CHCl_3$ (mol) | $CH_3OH$ (mol) | $CH_3CN$ (mol) | Reaction time (hr) | Reaction temperature | Yield (%) of compound C |
|---|---|---|---|---|---|---|---|---|---|---|
| 2 | 1 | 5.0 | - | - | 50 | 100 | - | 6.5 | Reflux | 52.5 |
| 3 | 1 | 1.5 | 1 ($HgCl_2$) | - | 60 | 120 | - | 2.0 | Reflux | 82.4 |
| 4 | 1 | 1.5 | 1 ($HgCl_2$) | 1 | 60 | 120 | - | 2.0 | Reflux | 92.8 |
| 5 | 1 | 1.0 | 1 ($HgCl_2$) | 1 | 50 | 100 | - | 1.0 | Reflux | 88.2 |
| 6 | 1 | 1.0 | 1 ($HgCl_2$) | 1 | 100 | - | - | 10.0 | Reflux | 79.5 |
| 7 | 1 | 1.5 | 1 ($CuCl_2$) | 1 | 50 | 100 | - | 1.0 | Reflux | 62.6 |
| 8 | 1 | 1.5 | 1.5 ($CuCl_2$) | 1 | 100 | - | 150 | 2.0 | Reflux | 45.2 |
| 9 | 1 | 1.5 | 1 ($CuBr_2$) | 1 | 100 | 200 | - | 1.0 | Reflux | 89.9 |

Example 10

(3α, 16α)-Eburunamenine-14(15H)-one 0-[2-hydroxy-3-[4-(2-methoxyphenyl)-1-piperazinyl]propyl]oxime

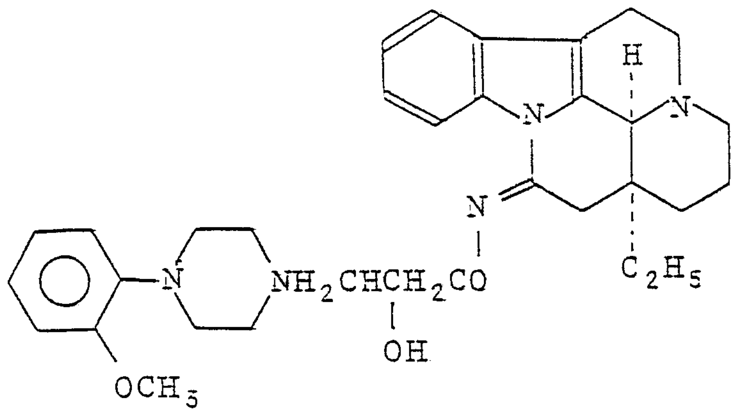

A mixture of (3α, 16α)-eburunamenine-14(15H)-thione (36.8mg, 0.119 mmol), 2-hydroxy-3-[4-(2-methoxyphenyl)-1-piperazinyl]propoxyamine (50.0mg, 0.178mmol), mercuric chloride (32,2mg, 0.119mmol), potasium carbonate (16.4MG, 0.119mmol), acetonitrile (1.00ml, 19.1mmol) and chloroform (1.00ml, 12.4mmol) was refluxed for one hour under vigorous stirring. The reaction mixture was worked up, as described in Example 1, to give 63.0mg (95.4%) of the desired compound as pale yellow oil.

Mass spectrum (m/z) : 558

NMR spectrum ($CDCl_3$) δ ppm :

0.90 (3H, t, J = 7.0Hz, $-CH_2CH_3$)

0.5 - 3.5 (24H, t, j = 7.0Hz, $-CH_2$ x 12)

3.90 (4H, broad s, $-OCH_3$ and $C_{3\alpha}$-H)

4.25 (4H, broad s, $-CH_2O$-and $>CH-OH$)

7.0 (4H, broad s, Ar-H)

7.1 - 7.7 (3H, broad s, Ar-H)

8.3 - 8.6 (1H, broad s, Ar-H)

IR spectrum ($\nu_{max}^{CHCl_3}$) $cm^{-1}$ :

3450 (OH), 3100 - 2800 (C-H), 1640, 1615 (C=C, C=N), 1505, 1460, 1240, 1030

**Claims**

1. A process for the preparation of an eburnamenine oxime derivative of the formula

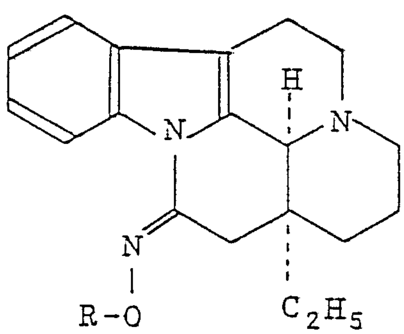

(I)

wherein R is an alkyl group, alkoxyalkyl group, oxylanylalkyl group, aralkyl group, aryl group, heteroaryl group,

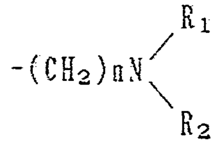 group or 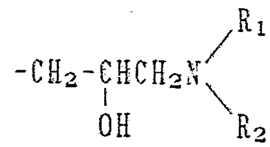 group,

each of $R_1$ and $R_2$ is same or different and represents hydrogen atom. an alkyl group having 1 to 4 carbon atoms, aryl group or aralkyl group, or $R_1$ and $R_2$ may form a substituted or unsubstituted heterocyclic radical together with the neighboring nitrogen atom, and n is an integer of 2 or 3,
which comprises a step of reacting eburnamenine thione of the formula

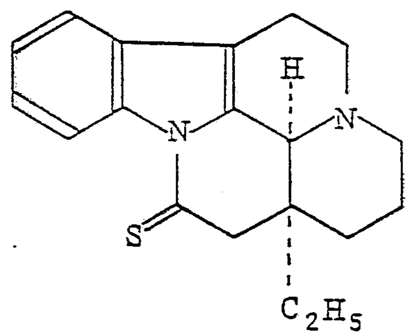

(II)

with a compound of the formula
$R\text{-}ONH_2$ (III)
wherein R represents the meaning as referred to.

2. A process as claimed in Claim 1, wherein said reaction is carried out in the presence of at least one of solvents selected from the group consisting of methanol, ethanol, isopropanol, tetrahydrofuran, dioxane, acetonitrile, dimethylsulfoxide, carbon tetrachloride, chloroform, methylene chloride, triethylamine. pyridine, benzene and toluene.

3. A process as claimed in Claim 1, wherein said reaction is carried out in the presence of at least one of compounds selected from the group consisting of mercuric chloride, cupric chloride, cupric bromide, manganese chloride, stannous chloride, mercuric acetate, cupric acetate, sodium hydrogen carbonate, sodium carbonate, potassium hydrogen carbonate, potassium carbonate, sodium acetate, potassium acetate, sodium hydrogen phosphate, potassium hydrogen phosphate, potassium phosphate, calcium carbonate, strontium carbonate, calcium acetate, strontium acetate, calcium phosphate and strontium phosphate.

4. A process as claimed in Claim 1, wherein said compound (II) is reacted with said compound (III) in a molar ratio within a range of 1 : 1 to 1 : 5.

5. A process as claimed in Claim 2, wherein said solvent is a mixture of chloroform and acetonitrile.